# EUROPEAN PATENT APPLICATION

(11) **EP 0 818 238 A1**
(43) Date of publication of application: **14.01.1998**
(21) Application number: 97900192.2
(22) Date of filing: 08.01.1997
(51) Int. Cl.: B01J 29/068, B01J 29/06, C07C 15/073, C07C 15/085

(54) **A CATALYST FOR PRODUCING ALKYL-BENZENE AND METHOD OF PRODUCING ALKYL-BENZENE USING THE CATALYST**

(30) Priority: 25.01.1996 CN 96116225; 25.01.1996 CN 96116227
(71) Applicant: CHINA PETROCHEMICAL CORPORATION, Beijing 100029 (CN); China Petrochemical Corporation Shanghai Research Institute of Petrochemical Technology, Shanghai 201208 (CN)
(72) Inventor: FANG, Yongcheng, Shanghai 201208 (CN); ZHOU, Bin, Shanghai 201208 (CN); DAI, Debin, Shanghai 201208 (CN); JIANG, Guixiu, Shanghai 201208 (CN); XIONG, Jin, Shanghai 201208 (CN)
(74) Representative: Woods, Geoffrey Corlett
(86) International application number: CN9700002
(87) International publication number: WO9726990

(57) **Abstract**

This invention provides a catalyst for producing alkyl-benzene. The catalyst of the invention comprises zeolite having molar ratio of SiO₂ to Al₂O₃ from 2 to 20, palladium supported on said zeolite and optional one or more metals selected from the group consisting of Ga, Ni, Co, Ag and Ir. This invention also relates to a process for producing alkyl-benzene by using said catalyst at the temperature from 150 °C to 250 °C and at the pressure from 0.7 to 4.0 Mpa, the molar ratio of benzene to olefins from 4 to 20.

## Description

### Field Of The Invention

The present invention relates to a catalyst and a process for preparing alkyl benzene, and in particular, to a catalyst containing zeolite supported with metal palladium and cocatalyst and a process employing the same for preparing isopropyl benzene and ethyl benzene.

### Background Of The Invention

Ethyl and isopropyl benzenes are important raw materials in organic chemical industry. For example, isopropyl benzene is a major intermediate compound in the production of phenol, acetone and α-methyl styrene, as well as an auxiliary of aviation petrol to increase the octane number. There are several catalysts for the synthesis of isopropyl benzene from propylene and benzene, such as the conventional catalysts crystalline and amorphous silica, boron trifluoride, alumina and phosphoric acid, aluminum trichloride, and solid phosphoric acid. However, the processes employing these catalysts suffer from such disadvantages as serious erosion of equipment, high capital cost and much waste effluents. In recent years, as deep studies on molecular sieves are carried out, new process for preparing isopropyl benzene with catalyst comprising zeolite attracts much attention. EP 0, 272, 830 disclosed a process utilizing Y zeolite molecular sieve to produce isopropyl benzene. Such a process overcomes the drawbacks of a process with conventional catalyst, though the catalyst has shortcomings, such as low stability and short service life ( about 700 hours of operation), low reaction space velocity ( only 0.10h⁻¹ based upon propylene ) and low output of the equipment.

### Disclosure Of The Invention

It is an object of the present invention to provide a new catalyst for preparing alkyl benzene, which achieves excellent activity, selectivity and stability, comprising
a) a zeolite with a molar ratio of silica to alumina of 2-20, and carried thereupon
b) metal palladium, and optionally one or more metal(s) selected from the group consisting of Ga, Ni, Co, Ag and Ir.

Another object of the present invention is to provide a process for preparing alkyl benzene, which comprises reacting olefin with benzene in liquid phase at a temperature of 150-250 °C and a polar ratio of benzene to olefin of 4-20 under a pressure of 0.7-4.0Mpa in the presence of a catalyst comprising
a) a zeolite with a molar ratio of silica to alumina of 2-20, and carried thereupon
b) metal palladium, and optionally one or more metal(s) selected from the group consisting of Ga, Ni, Co, Ag and Ir,

Said process achieving an improved output and a long service life of the catalyst.

### Preferred Embodiment Of The Invention

In one aspect, the present invention provides a catalyst for preparing alkyl benzene, comprising
a) a zeolite with a molar ratio of silica to alumina of 2-20, and carried thereupon
b) metal palladium, and optionally one or more metal(s) selected from the group consisting of Ga, Ni, Co, Ag and Ir.

The zeolite used herein can be Y zeolite, β zeolite, mordenite and/or ZSM-5 zeolite. The molar ratio of silica to alumina of the zeolite used herein is 2-20, preferably 2-8.

In a preferred embodiment of the invention, the metal palladium is present in an amount of 0.003-3.0%, preferably 1-1.5%, by weight, based upon the zeolite. The catalyst can further optionally contains 0-3.0%, preferably 0.5-2.0%, by weight of one or more metal(s) selected from the group consisting of Ga, Ni, Co, Ag and Ir.

The present catalyst can be prepared as follows:
a) Exchanging a zeolite with a molar ratio of silica to alumina of 2-20 with acid and ammonium salt until a total amount of sodium ion and potassium ion in the zeolite backbone reaches below 0.1%, then drying; and
b) impregnating the zeolite treated as above with palladium metal and optionally Ga, Ni, Co, Ag and/or Ir.

The acid useful in the treatment of the zeolite can be conventional acid, Such as HCl, H₂SO₄, HNO₃ or H₃PO₄. The aimmonium salt useful in the treatment of the zeolite can be conventional ammonium salt, such as NH₄Cl, (NH₄)₂SO₄ or (NH₄)₃PO₄.

The present catalyst is suitable for preparing alkyl benzene, especially isopropyl benzene and ethyl benzene.

In another aspect, the present invention provides a process for preparing alkyl benzene, comprising carrying out reaction in liquid phase at a temperature of 150-250°C, preferably 180-210 °C , and at a molar ratio of benzene to olefin of 4-20 under a pressure of 0.7-4.0Mpa, preferably 2.0-3.5Mpa, in the presence of the present catalyst.

The process of the present invention is suitable for preparing alkyl benzene with a lower carbon number alkyl group, for example, alkyl benzenes with an alkyl group of no more than 6 carbon atoms, and is especially suitable for preparing isopropyl benzene and ethyl benzene.

The process of the invention is usually carried out in a fixed bed mode.

The catalyst of the present invention achieves good activity, selectivity and stability, and the process employing the catalyst of tile invention achieves high liquid phase space velocity and high output.

The present invention will be described in more detail by the following nonlimiting Examples.

### Examples 1-7

Examples 1-7 relates to catalyst preparation.

### Example 1

100g NaY zeolite powder with a molar ratio of silica to alumina of 2.0 was exchanged with acid, then with ammonium salt , thereby the Na⁺ amount in the zeolite being brought below 0.1%. The zeolite treated as above was impregnated with PdCl₂ solution to reach an amount of metal palladium of 0.09% by weight. After drying and calcining, it was ground, kneaded with alumina ( Y zeolite: alumina = 90:10 by weight ) and extruded into ⌀1.6mm pellets.

### Comparative Examples 1

100g NaY zeolite powder with a molar ratio of silica to alumina of 2.0 is exchanged with acid, then with ammonium salt , thereby the Na⁺ amount in the zeolite being brought below 0.1%. After drying, it was ground, kneaded with alumina ( Y zeolite: alumina = 90:10 ), and extruded into ⌀1.6mm pellets.

### Example 2

100g NaY zeolite powder with a molar ratio of silica to alumina of 5.0 was exchanged with acid, then with ammonium salt , thereby the Na⁺ amount in the zeolite being brought below 0.1%. The zeolite treated as above was impregnated with PdCl₂ solution, Ni(NO₃)₂ solution and Co(NO₃)₂ solution until the contents of Pd, Ni and Co reached 0.003%, 0.5% and 0.2% by weight respectively. After drying and calcining, the zeolite was ground, kneaded with alumina ( Y zeolite: alumina = 90:10 ), and extruded into ⌀1.6mm pellets.

### Example 3

100g NaY zeolite powder with a molar ratio of silica to alumina of 3.0 was exchanged with acid, then with ammonium salt, thereby the Na⁺ amount in the zeolite being brought below 0.1%. The zeolite treated as above was impregnated with PdCl₂ solution, Ga(NO₃)₂ solution, Ni(NO₃)₂ solution, AgNO₃ solution and Ir(NO₃)₂ solution until the amounts of Pd, Ga, Ni, Ag and Ir were 0.8%, 0.1%, 0.3%, 0.1% and 0.2% by weight respectively. After drying and calcining, the zeolite was ground, kneaded with alumina ( Y zeolite: alumina = 90:10 ), and extruded into ⌀1.6mm pellets.

### Example 4

The catalyst was prepared as in Example 3, except that the zeolite employed was β zeolite with a molar ratio of silica to alumina of 14.0, and the impregnated metals were Pd 1.2%, Ga 0.1%, Ni 0.3%, Co 0.7% and Ag 0.2% by weight.
The zeolite was kneaded with alumina ( Y zeolite: alumina = 90:10 ), and extruded into ⌀1.6mm pellets.

### Example 5

The catalyst was prepared as in Example 3, except that the zeolite employed was mordenite with a molar ratio of silica to alumina of 15, and the impregnated metals were Pd 1.5%, Ga 0.1%, Ni 0.8%, Co 0.2% and Ir 0.1% by weight.
The zeolite was kneaded with alumina ( Y zeolite: alumina = 90:10 ), and extruded into ⌀1.6mm pellets.

### Example 6

The catalyst was prepared as in Example 3, except that the zeolite employed was ZSM-5 zeolite with a molar ratio of silica to alumina of 20, and the impregnated metals were Pd 2.0%, Ni 0.4%, Co 0.2%, Ag 0.1% and Ir 0.1% by weight. The zeolite was kneaded with alumina ( Y zeolite: alumina = 90:10 ), and extruded into ⌀1.6mm pellets.

### Example 7

100g NaY zeolite powder with a molar ratio of silica to alumina of 5.0 was exchanged with acid, then with ammonium salt, thereby the Na⁺ content of the zeolite being brought below 0.1%. The zeolite treated as above was impregnated with PdCl₂ solution, Ni(NO₃)₂ solution and Co(NO₃)₂ solution until the metal contents reach Pd 0.95%, Ni 0.5% and Co 0.75% respectively. After drying and calcining, the zeolite was ground, kneaded with alumina ( Y zeolite: alumina = 90:10 ), and extruded into ⌀1.6mm pellets.

### Examples 8-14

Examples 8-14 relates to catalyst evaluation

### Example 8

21g of the catalyst of Example 1 was loaded into a fixed bed reactor, through which benzene and propylene were passed to effect reaction at 195 °C and 2.6Mpa, wherein benzene and propylene feeding rates were 468g/h and 42g/h respectively. Based upon propylene, the weight space velocity of liquid was 2.0h⁻¹. After 1000 hours' time of reaction, no propylene was detected in the reaction product, without decreasing of the catalyst activity and selectivity The selectivity to isopropyl benzene was 98.0% ( based upon propyl groups).

### Comparative Example 2

21g of the catalyst of Comparative Example 1 was loaded into a fixed bed reactor, through which benzene and propylene were passed to effect reaction at 195 °C and 2.6Mpa, wherein benzene and propylene feeding rates were 468g/h and 42g/h respectively. Based upon propylene, tile weight space velocity of liquid was 2.0h⁻¹. After 100 hours' time of reaction, analysis showed that the selectivity to isopropyl benzene was 97.8%. After 140 hours' time, there was propylene detected in the reaction product, which meaned that the catalyst was deactivated.

### Example 9

21g of the catalyst of Example 2 was loaded into a fixed bed reactor, through which benzene and propylene were passed to effect reaction at 182 °C and 2.0Mpa, wherein benzene and propylene feeding rates were 702g/h and 42g/h respectively. Based upon propylene, the weight space velocity of liquid was 2.0h⁻¹. After 1000 hours' time of reaction, no propylene was detected in the reaction product, without decreasing of the catalyst activity and selectivity. The selectivity to isopropyl benzene was 98.7%.

### Example 10

21g of the catalyst of Example 3 was loaded into a fixed bed reactor, through which benzene and propylene were passed to effect reaction at 210 °C and 2.6Mpa, wherein benzene and propylene feeding rates were 702g/h and 42g/h respectively. After 1000 hours' time of reaction, no propylene was detected in the reaction product, without decreasing of the catalyst activity and selectivity. The selectivity to isopropyl benzene was 97.1%.

### Example 11

21g of the catalyst of Example 4 was loaded into a fixed bed reactor, and the reaction was carried out as in Example 10. After 1000 hours' time of reaction, no propylene was detected in the reaction product, without decreasing of the catalyst activity and selectivity. The selectivity to isopropyl benzene was 96.4%.

### Example 12

84g of the catalyst of Example 5 was loaded into a fixed bed reactor, and the reaction was carried out as in Example 10. After 45 hours' time of reaction, no propylene was detected in the reaction product, without decreasing of the catalyst activity and selectivity. The selectivity to isopropyl benzene was 95.2%.

### Example 13

168g of the catalyst of Example 6 was loaded into a fixed bed reactor, and the reaction was carried out as in Example 10. After 20 hours' time of reaction, there was no propylene detected in the product, namely, the activity and selectivity of the catalyst did not decreased. The selectivity to propyl benzene was 96.9%.

### Example 14

21g of the catalyst of Example 7 was loaded into a fixed bed reactor, through which benzene and ethylene were passed to effect reaction at 250 °C and 4.0Mpa, wherein benzene and ethylene feeding rates were 400g/h and 10.5g/h respectively. Based upon ethylene, the weight space velocity of liquid was 0.5h⁻¹. After 1000 hours' time of reaction, no ethylene was detected in the reaction product, without decreasing of the catalyst activity and selectivity. The selectivity to ethyl benzene was 90% ( based upon ethyl group ).

## Claims

1. An alkylating catalyst for preparing alkyl benzene, comprising
a) a zeolite with a molar ratio of silica to alumina of 2-20, and carried thereupon
b) metal palladium, and optionally one on more metal(s) selected from the group consisting of Ga, Ni, Co, Ag and Ir.

2. A catalyst of claim 1, comprising 0.003%-3.0% by weight of metal palladium and 0-3.0% by weight of one or more metal(s) selected from the group consisting of Ga, Ni, Co, Ag and Ir.

3. A catalyst of claim 1, wherein said zeolite is a zeolite with a molar ratio of silica to alumina of 2-8.

4. A catalyst of claim 1, wherein said zeolite is Y zeolite, β zeolite, mordenite and/or ZSM-5 zeolite.

5. A catalyst of claim 3, wherein said zeolite is Y zeolite.

6. A catalyst of claim 1, comprising 1-1.5% by weight of metal palladium.

7. A catalyst of claim 1, comprising 0.5-2% by weight of one or more metal(s) selected from the group consisting of Ga, Ni, Co, Ag and Ir.

8. A process for preparing alkyl benzene, comprising reacting olefin with benzene in liquid phase at a reaction temperature of 150-250 °C and a reaction pressure of 0.7-4.0Mpa and a molar ratio of benzene to olefin of 4-20 in the presence of a catalyst comprising
a) a zeolite with a molar ratio of silica to alumina of 2-20; and carried thereupon
b) metal palladium, and optionally one or more metal(s) selected from the group consisting of Ga, Ni, Co, Ag and Ir.

9. A process of claim 8, wherein said alkyl benzene is isopropyl benzene and said olefin is propylene.

10. A process of claim 8, wherein said alkyl benzene is ethyl benzene and said olefin is ethylene.

11. A process of claim 8, wherein the reaction temperature is 180-210 °C.

12. A process of claim 8, wherein the reaction pressure is 2.0-3.5Mpa.
